# EUROPEAN PATENT SPECIFICATION

(11) **EP 1 027 602 B1**
(45) Date of publication and mention of the grant of the patent: **26.10.2005**
(21) Application number: 98937775.9
(22) Date of filing: 31.07.1998
(51) Int. Cl.: G01N 33/487, G01N 27/28, C12Q 1/00

(54) **A METHOD OF DETERMINING THE CONCENTRATION OF AN ANALYTE EMPLOYING A BIOELEMENT AND A TRANSDUCER AND A SMALL-VOLUME DEVICE FOR USE IN THE METHOD**
VERFAHREN ZUR BESTIMMUNG DER KONZENTRATION EINES ANALYTEN UNTER VERWENDUNG EINES BIOELEMENTES UND EINEN TRANSDUZER, UND EINE VORRICHTUNG MIT EINEM KLEINEN VOLUMEN ZUR VERWENDUNG IM VERFAHREN
PROCEDE POUR DETERMINER LA CONCENTRATION D'UN ANALYTE A L'AIDE D'UN BIOELEMENT ET D'UN TRANSDUCTEUR, ET UN DISPOSITIF A VOLUME PETIT UTILISE DANS LE PROCEDE

(30) Priority: 08.08.1997 IT PD970183
(43) Date of publication of application: 16.08.2000
(73) Proprietor: Nika S.r.l., 35010 Limena (PD) (IT)
(72) Inventor: SCARPA, Maria, I-30122 Sant'Elena (IT)
(74) Representative: Cantaluppi, Stefano
(86) International application number: PCT/IT1998/000224
(87) International publication number: WO 1999/008106

(56) References cited:
- EP-A- 0 244 326
- EP-A- 0 798 561
- WO-A-94/23295
- FR-A- 2 692 357
- US-A- 4 999 284
- US-A- 5 520 787
- MARK A. ARNOLD, ET AL.: "JACK BEAN MEAL AS BIOCATALYST FOR UREA BIOSENSORS" BIOTECHNOLOGY LETTERS, vol. 6, no. 5, 1984, pages 313-318, XP002089475 cited in the application

## Description

### Technical field

The present invention relates to a method of determining the concentration of an analyte with the use of a bioelement according to the preamble to the main claim.

The invention also relates to a device for determining the concentration of an analyte in accordance with the method of the invention.

### Technological background

Methods of determining the concentration of a compound, hereinafter referred to as an analyte, are of particular interest in the biomedical, industrial, environmental and agricultural/food fields. These methods provide for the use of biomolecules such as, for example, enzymes, antibodies, cells or the like, which can interact specifically with the analyte the concentration of which is to be determined. The interaction, which may be chemical, chemico-physical or physical, is such as to bring about a change in the characteristics of the analyte-bioelement system which can be suitably converted into a signal, for example, an electrical signal, which can be correlated with the concentration of the analyte. These methods provide for the use of transducers for detecting the above-mentioned changes and for converting them (with amperometric, potentiometric, conductometric, piezo-electric, optical, thermal transducers, etc.) into signals, normally electrical signals, which can be suitably amplified and processed, as well as displayed and/or recorded.

Amongst the known methods which provide for the use of analyte-bioelement systems (otherwise known as biosensors), reference is made to those described in the following publications:
- J. Mor, R. Guarnaccia (1977) Analytical Biochemistry 79, 319-328;
- D. Francis, H. Hill, W. Aston, W. Higgins, E. Plotkin, E. Scott, A. Turner (1984) Analytical Chemistry 56, 667-671;
- M. Arnold, S. Glazier (1984) Biotech. Letters 6, 313-328;
- A. Turner, I. Karube, G. Wilson, editors (1989) "Biosensors. Fundamentals and applications" Oxford University Press, 1-700;
- T. Ruzgas, J, Emneus, L. Gorton, G. Marko-Varga (1995) Analytica Chimica Acta 311, 245-253.

According to known methods, the concentration of the analyte is generally determined on the basis of a measurement of the rate of analyte-bioelement interaction, that is, of the rate of transfer of the analyte to the bioelement. These rates depend upon factors such as the activity of the bioelement, the temperature, the viscosity and the pH, as well as on the nature and physical characteristics of the matrix immobilizing the bioelement. This necessitates the production of a calibration curve correlating the signals generated by the transducer with the concentration of the analyte for each measurement or set of measurements of the analyte concentration. Although the use of these analytical methods often involves overall costs lower than those of conventional analytical instrumentation, it is thus limited by the instability of the bioelement, by the cost thereof, and by the need for accurate calibrations.

### Description of the invention

The problem upon which the present invention is based is that of providing a method and a device for determining the concentration of an analyte with the use of a bioelement which overcomes all of the problems complained of with reference to the prior art mentioned.

This problem is solved by the invention by means of a method and a device as defined in the claims for determining the concentration of an analyte with the use of a bioelement according to the following claims.

### Brief description of the drawings

The characteristics and the advantages of the invention will become clearer from the following detailed description of a preferred embodiment described by way of non-limiting example, with reference to the appended drawings, in which:
Figure 1 is a schematic plan view of a device formed in accordance with the invention,
Figure 2 is a section taken on the line II-II of Figure 1.

### Preferred method of implementing the invention

With reference to the aforementioned drawings, a device for determining the concentration of an analyte with the use of a bioelement according to the present invention is generally indicated 1.

The device 1 comprises a chamber 2 for holding the sample in which the analyte whose concentration is to be determined is present. The chamber 2 is defined by a first surface and a second surface disposed opposite one another and indicated 3 and 4 respectively, and by a further lateral surface 5.

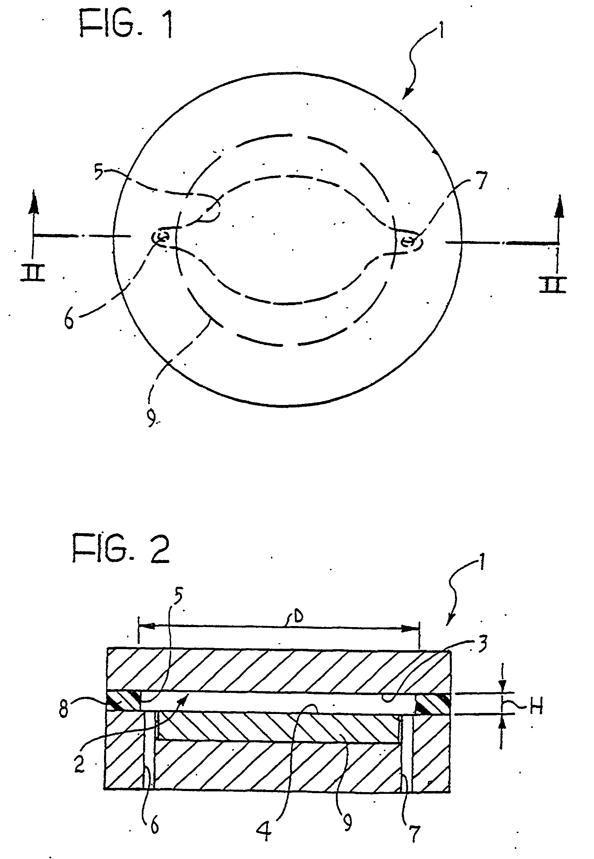

Holes formed in the surface 4 put the chamber 2 into communication with a first, inlet capillary duct 6 and a second, outlet capillary duct 7 for admitting the sample containing the analyte to be analyzed to the chamber 2 and discharging it therefrom, respectively. The chamber 2 is formed by superimposing two plate-like elements defining the surfaces 3 and 4 with the interposition of a film seal 8 of which the thickness, indicated H in Figure 2,defines the distance between the aforementioned surfaces 3 and 4. A hole in the seal 8 defines the lateral surface 5 of the chamber and has a shape which is elongate in plan towards the inlet and outlet openings of the chamber, as shown in Figure 1, in which D indicates the length of the chamber in the direction in which it is elongate.

The depth H of the chamber 2, which is equal to the distance between the surfaces 3 and 4, is selected so as to be equal to or less than 500 micrometres and, advantageously, between 2 and 200 micrometres. A preferred selection for the depth H is between 20 and 100 micrometres.

The selected ratio between the length D and the depth H (the distance between the surfaces 3 and 4) of the chamber 2 is selected so as to be between 10 and 2000 and is preferably between 50 and 500.

The surface 4 is preferably defined by the active surface of a transducer 9 shown only schematically in Figure 1, which can detect the chemical, chemico-physical, or physical changes due to the interaction of the analyte with a preselected bioelement introduced into the chamber 2, and can convert these changes into a measurable physical magnitude correlated with the concentration of the analyte to be determined, as will be explained further in the following description. The transducer may be electrochemical (amperometric, potentiometric, conductometric), optical, piezo-electric, thermal, etc., according to the physical magnitude to be measured. The surface 4 may also be transparent to the signal used for measuring the bioelement-analyte interaction, to permit detection by an external transducer.

The bioelement selected for interacting with the analyte is arranged on the surface 3 opposite the transducer 9. The bioelement may be immobilized on the surface 3 chemically and/or physically. Alternatively, the bioelement may be introduced into the chamber 2 directly with the sample containing the analyte to be analyzed. As a further alternative, the bioelement may be immobilized on the same surface as the transducer or the two surfaces may form portions of the transducer.

The plate-like element defining the surface 3 on which the bioelement can be immobilized is associated with the device 1 interchangeably and may, for example, be in the form of a glass plate.

The concentration of the analyte present in the sample is determined the following manner. First of all, the sample containing the analyte to be analyzed is introduced into the chamber 2 through the inlet duct 6 by capillarity or simply by injection, possibly with the addition of a buffer, a mediator, and/or other substance capable of facilitating the interaction with the transducer 9. The interaction of the analyte with the bioelement, which may be disposed on the surface 3 or introduced into the chamber 2 directly with the sample, brings about a change in the chemical, chemico-physical and/or physical characteristics of the analyte-bioelement system. At least one of these characteristics changes over time from the moment when the analyte-bioelement interaction starts up to a final moment at which all of the analyte has interacted with the bioelement. The characteristics of the analyte-bioelement system are detected and converted by the transducer 9 into a signal variable within the period of time between the aforementioned starting and final moments in dependence on the activity of the bioelement and tending towards a constant value or towards zero after the analyte-bioelement interaction has been completed.

If the sample in which the analyte is present contains other compounds which can interact directly or indirectly with the transducer, the signal detected in the absence of the bioelement is subtracted from the signal detected during a measurement carried out in the presence of the bioelement so as to isolate solely the signal generated as a result of the analyte-bioelement interaction.

The large ratio between the length D and the depth H of the chamber, which is representative of the surface area:volume ratio of the chamber, together with the small depth H thereof, enables the analyte to diffuse rapidly between the surfaces 3 and 4 of the chamber 2 within periods of the order of one minute or less.

It should be noted that the measurement is carried out in conditions in which the solution to be analyzed is substantially still, that is, making use solely of the motion of the molecular diffusion of the analyte-bioelement system and not in conditions providing for a flow and/or stirring of the aforesaid solution.

A high D:H ratio and a small depth H also achieve a high concentration of the bioelement within the chamber 2 and consequently a high activity of the bioelement per unit volume, even with a small number of molecules of the bioelement (for example, it suffices to provide a monomolecular layer of the selected bioelement on the surface 3). This means that all of the molecules of the analyte present in the chamber 2 interact with the bioelement within extremely short periods and the products of the interaction diffuse rapidly over the surface of the transducer with diffusion times of the order of one minute or less. The quantity of bioelement in the chamber 2 and the diffusion times between the surfaces 3, 4 defining the chamber are in any case such as to ensure that all of the analyte interacts with the bioelement within periods of the order of a few minutes.

After all of the analyte has interacted with the bioelement, the signal detected by the transducer tends towards a value which is substantially constant within the experimental error. The magnitude measured, or its variation, is proportional to the initial concentration of the analyte, as will become clearer from the following description of the examples. The measurement of this magnitude is therefore independent of the activity of the bioelement.

With the device of the present invention, the concentration of the analyte is determined according to the nature of the analyte, the nature of the bioelement, and the type of analyte-bioelement interaction.

For example, to determine the concentration of the sulphite ion (analyte) with the use of sulphite oxidase as the bioelement, in the presence of an oxidizing agent such as ferricyanide, the surface 4 is constituted by a thin sheet of a noble metal (Au, Pt, etc.), the electrical potential of which, measured relative to a reference electrode, for example, in Ag-AgCl, varies in dependence on the [ferricyanide]. [ferrocyanide] molar ratio, where the ferrocyanide represents the product of the reaction of the sulphite-sulphite oxidase-ferricyanide system, since the sulphite oxidase (the bioelement) is immobilized on the surface 3 and the ferricyanide (mediator) is in solution. Alternatively, a glass electrode may be used instead of the noble metal plate and the change in pH which accompanies the aforementioned reaction may be measured. In both cases, the transducer used is a potentiometric transducer.

To determine the concentration of an antigen (analyte), for example, human albumin, with the use, as a bioelement, of an antibody which can bind the albumin highly specifically, the transducer used comprises a piezo-electric crystal on the surface 4 of which the aforesaid antibodies are immobilized. The concentration of the analyte is determined by measurement of the change in the resonance frequency of the piezo-electric crystal due to the change in the mass of the piezo-electric crystal-antibody system as a result of the binding of all of the antigen to the immobilized antibody. In this case, the transducer selected is a piezo-electric transducer.

To determine the concentration of an analyte which brings about the emission of one or more photons during the interaction with the bioelement, for example, to determine ATP (adenosine triphosphate) with the luciferin-luciferase system, the surface 4 is constituted by that of a phototube (transducer) or that of a medium which is optically transparent to the photons emitted, or in any case such as to allow the photons to interact with the transducer. The concentration of the analyte is determined by measurement of the number of photons emitted during the interaction of all of the ATP with the luciferase (bioelement) in the presence of an excess of luciferin, the luciferase being immobilized on the surface 3. In this case, the transducer used is an optical transducer.

Two examples of the determination of the concentration of analytes by the method and the device of the present invention are given below.

### Example 1

Determination of the concentration of glucose with the use of glucose oxidase and peroxidase enzymes and with the use of an amperometric transducer.

In this application the bioelements (glucose oxidase and peroxidase) were immobilized covalently on the surface of a suitably prepared glass plate which defined one surface of the chamber. The quantities of glucose oxidase and peroxidase immobilized were such as to ensure that all of the glucose present in the chamber was converted within periods of less than one minute. The opposite surface was defined by a transducer comprising a vitreous graphite electrode constituting the working electrode of a voltammetric circuit which further comprised a reference electrode and a contro-electrode. The depth H of the chamber was 100 micrometres with a chamber volume of 2 microlitres. The sample to be analyzed, which was constituted by a glucose solution, was diluted with a concentrated pH 6.5 sodium phosphate solution containing 1mM (millimolar) hydroquinone, to give a 0.1mM glucose solution. After the solution thus obtained had been introduced into the chamber, a potential of -300 mV relative to the reference electrode in Ag/AgCl was applied to the working electrode and the electrical current flowing in the electrochemical circuit was measured until the value of the current due to the reduction of the quinone formed during the oxidation of the glucose via glucose oxidase-peroxidase, fell substantially to zero, that is, until all of the quinone which was formed in an equivalent quantity to the glucose had been reduced. The electrical-current signal generated was integrated over time and the quantity of electrical charge thus measured was 39 microcoulombs, which value corresponds, within the experimental error, to the value calculated theoretically on the basis of the glucose present in the chamber.

### Example 2

Determination of the concentration of urea by means of the urease enzyme with the use of a conductometric transducer.

In this application, the opposed surfaces of the chamber were defined by two flat electrodes of a conductometric circuit. The solution to be analyzed contained a 1mM concentration of urea (analyte). After the conductivity of the solution to be analyzed had been measured in conditions similar to those of the previous example, a small quantity of a solution containing the urease enzyme (this solution was equal in quantity to one hundredth of the volume of the solution of analyte and the final concentration of the enzyme in the chamber was such as to enable all of the urea to be converted within a period of less than one minute) was added to the solution containing the analyte and the increase in the conductivity of the solution due to the conversion of all of the urea into ammonium ions and bicarbonate ions was measured. The increase in conductivity measured, which was 5.2 mSiemens, corresponded approximately, within the experimental error, to the value calculated theoretically on the basis of the initial concentration of urea in the chamber.

The invention thus solves the problem set, achieving many advantages in comparison with known solutions.

First of all, with the device of the invention, all of the molecules of analyte present in the volume of the chamber interact with the bioelement with extremely short diffusion times between the surfaces of the chamber so that all of the analyte interacts with the bioelement within periods of the order of one minute or less.

Moreover, since the signal generated by the transducer, which is proportional to the concentration of the analyte, is measured after all of the analyte has interacted with the bioelement, even very marked variations in the activity of the bioelement do not affect the change in the signal detected and it is therefore not necessary to calibrate the instrumentation for each measurement or set of measurements to be made.

Another advantage is that, owing to the extremely small volume of the chamber of the device of the invention, the quantity of bioelement necessary to determine the concentration of the analyte is greatly reduced, consequently reducing analysis costs.

A further advantage connected with the small volumes of the chamber of the device of the invention is that, with the same plate on which the bioelement is disposed, it is possible to perform several analyte determinations relative to the bioelement immobilized.

Moreover, the interchangeability of the plate on which the bioelement is disposed enables the concentration of different analytes to be measured according to the type of bioelement selected.

Not the least advantage is that, by virtue of the dimensional ratios between the length and the depth of the chamber of the device of the invention, the analyte-bioelement interaction and the diffusion of the analyte between the surfaces take place without the need to stir the solution containing the sample to be analyzed, thus eliminating possible signal variations due to any variations in the stirring speed.

## Claims

1. A method of determining the concentration of an analyte with the use of a bioelement, comprising the steps of:
- introducing into a chamber (2) comprising a first surface and a second surface (3, 4) disposed opposite one another, a sample containing the analyte, and a quantity of a bioelement able to interact specifically with the analyte, bringing about a change in the chemical, physical and/or chemico-physical characteristics of the analyte-bioelement system,
- providing transducer means (9) on at least one of the surfaces (3, 4), which converts the analyte-bioelement interaction into a physical magnitude which is correlative with the concentration of the analyte, **characterized in that**
- the analyte-bioelement interaction takes place within the chamber (2) having a depth (H), defined as the distance between the surfaces (3, 4) which is selected so as to be less than or equal to 500 micrometres,
- **in that** the quantity of the selected bioelement is such as to ensure that all of the analyte interacts with the bioelement and
- **in that** the concentration of the analyte is determined after all of the analyte has interacted with the bioelement when the magnitude tends to adopt an approximately constant value which is proportional to the concentration of the analyte and is also independent of the activity of the bioelement.

2. A method of determining the concentration of an analyte with the use of a bioelement, comprising the steps of:
- introducing into a chamber (2) comprising a first surface and a second surface (3, 4) disposed opposite one another, a sample containing the analyte,
- providing on at least one of the surfaces (3, 4) a quantity of a bioelement able to interact specifically with the analyte, bringing about a change in the chemical, physical and/or chemico-physical characteristics of the analyte-bioelement system,
- providing transducer means (9) on at least one of the surfaces (3, 4) which converts the analyte-bioelement interaction into a physical magnitude which is correlative with the concentration of the analyte, **characterized in that**
- the analyte-bioelement interaction takes place within the chamber (2) having a depth (H), defined as the distance between the surfaces (3, 4) which is selected so as to be less than or equal to 500 micrometres,
- **in that** the quantity of the selected bioelement is such as to ensure that all of the analyte interacts with the bioelement and
- **in that** the concentration of the analyte is determined after all of the analyte has interacted with the bioelement when the magnitude tends to adopt an approximately constant value which is proportional to the concentration of the analyte and is also independent of the activity of the bioelement.

3. A method according to Claim 1 or Claim 2, in which the change in the chemical, physical and/or chemico-physical characteristics of the analyte-bioelement system is converted into an electrical-current signal and the physical magnitude represents the total electrical charge obtained by integration of the amplitude of the electrical-current signal within the period of time between the moment at which the analyte-bioelement interaction starts and the moment at which the change tends towards zero or the magnitude tends towards a substantially constant value, the total quantity of electrical charge being proportional to the concentration of the analyte.

4. A method according to Claim 1 or Claim 2, in which the magnitude is represented by the electrical conductivity of a solution containing the analyte-bioelement system, the electrical conductivity being measured after all of the analyte has interacted with the bioelement when the conductivity tends towards a substantially constant value; the conductivity value measured being proportional to the concentration of the analyte.

5. A device for determining the concentration of an analyte with the use of a bioelement according to the method of any one of Claims 1 to 4, comprising a chamber for holding a analyte-bioelement system and transducer means associated with the chamber for detecting the change in the chemical, physical and/or chemico-physical characteristics of the analyte-bioelement system and converting the changes into a physical magnitude which can be correlated with the concentration of the analyte, the chamber comprising a first surface on which at least a portion of the transducer means is disposed, and an opposed second surface, the depth of the chamber, which is equal to the distance between the surfaces, being less than or equal to 500 micrometres, a bioelement being disposed on at least one of the surfaces, **characterized in that** comprises a plate-like element associated interchangeably with the device, and defining the surface on which the bioelement is disposed.

6. A device according to Claim 5, in which the depth is between 2 and 200 micrometres and is preferably selected between 20 and 100 micrometres.

7. A device according to Claim 5 or Claim 6, in which the ratio between the length and the depth of the chamber which is equal to the distance between the surfaces is between 50 and 500.

8. A device according to any one of Claims 5 to 7, in which at least one of the surfaces is defined by the transducer.

9. A device according to any one of Claims 5 to 8, in which the bioelement is disposed on the opposite surface to the transducer.

10. A device according to any one of Claims 5 to 8, in which the bioelement is disposed on the surface defined by the transducer.

11. A device according to Claim 9, in which the bioelement is immobilized chemically and/or physically on the surface opposite the transducer.

12. A device according to any one of Claims 5 to 11, in which the surfaces are separated by a seal defining a lateral surface of the chamber, the device comprising at least one duct for the inlet of the solution containing the analyte to the chamber and for its outlet therefrom, respectively.

13. A device according to Claim 12, in which the ducts are capillary ducts.

14. A device according to Claim 7, in which the area of the surface of the chamber defined by the transducer is substantially equal to the area of the opposite surface on which the bioelement is disposed.

15. A device according to one or more of Claims 5 to 14, in which the transducer means are potentiometric.

16. A device according to one or more of Claims 5 to 14, in which the transducer means are piezo-electric.

17. A device according to one or more of Claims 5 to 14, in which the transducer means are optical.

18. A device according to one or more of Claims 5 to 14, in which the transducer means are amperometric or conductometric.

## Patentansprüche

1. Verfahren zur Bestimmung der Konzentration eines Analyten unter Verwendung eines Bioelements, umfassend folgende Schritte:
- Einbringen einer Probe, die den Analyten enthält, und einer Menge eines Bioelements, die in der Lage ist, insbesondere mit dem Analyten in Wechselwirkung zu treten, die eine Änderung der chemischen, physikalischen und/oder chemo-physikalischen Eigenschaften des Systems Analyt- Bioelement bewirkt, in eine Kammer (2), die eine erste Oberfläche und eine zweite Oberfläche (3, 4) aufweist, die einander gegenüber liegen,
- Bereitstellen von Übertragermitteln (9) auf wenigstens einer der Oberflächen (3, 4), die die Analyt- Bioelement- Wechselwirkung in eine physikalische Größe umwandeln, die mit der Konzentration des Analyten korreliert, **dadurch gekennzeichnet, dass**
- die Analyt- Bioelement- Wechselwirkung innerhalb der Kammer (2) stattfindet, die eine Tiefe (H) aufweist, die als Abstand zwischen den Oberflächen (3, 4) definiert ist, und die mit 500 µm oder weniger gewählt wird,
- die Menge des ausgewählten Bioelements so gewählt wird, dass sichergestellt ist, dass die gesamte Analytmenge mit dem Bioelement in Wechselwirkung tritt, und
- die Konzentration des Analyten bestimmt wird, nachdem die gesamte Analytmenge mit dem Bioelement in Wechselwirkung getreten ist, wenn die physikalische Größe dazu tendiert, einen angenähert konstanten Wert anzunehmen, der proportional zu der Konzentration des Analyten und dabei unabhängig von der Aktivität des Bioelements ist.

2. Verfahren zur Bestimmung der Konzentration eines Analyten unter Verwendung eines Bioelements, umfassend folgende Schritte:
- Einbringen einer Probe, die den Analyten enthält, in eine Kammer (2), die eine erste Oberfläche und eine zweite Oberfläche (3, 4) aufweist, die einander gegenüber liegen,
- Bereitstellen auf wenigstens einer der Oberflächen (3, 4) einer Menge eines Bioelements, die in der Lage ist, insbesondere mit dem Analyten in Wechselwirkung zu treten, die eine Änderung der chemischen, physikalischen und/oder chemophysikalischen Eigenschaften des Systems Analyt-Bioelement bewirkt,
- Bereitstellen von Übertragermitteln (9) auf wenigstens einer der Oberflächen (3, 4), die die Analyt- Bioelement- Wechselwirkung in eine physikalische Größe umwandeln, die mit der Konzentration des Analyten korreliert, **dadurch gekennzeichnet, dass**
- die Analyt- Bioelement- Wechselwirkung innerhalb der Kammer (2) stattfindet, die eine Tiefe (H) äufweist, die als Abstand zwischen den Oberflächen (3, 4) definiert ist, und die mit 500 µm oder weniger gewählt wird,
- die Menge des ausgewählten Bioelements so gewählt wird, dass sichergestellt ist, dass die gesamte Analytmenge mit dem Bioelement in Wechselwirkung tritt, und
- die Konzentration des Analyten bestimmt wird, nachdem die gesamte Analytmenge mit dem Bioelement in Wechselwirkung getreten ist, wenn die physikalische Größe dazu tendiert, einen angenähert konstanten Wert anzunehmen, der proportional zu der Konzentration des Analyten und dabei unabhängig von der Aktivität des Bioelements ist.

3. Verfahren nach Anspruch 1 oder 2, wobei die Änderung der chemischen, physikalischen und/oder chemo-physikalischen Eigenschaften des Systems Analyt- Bioelement umgesetzt wird in ein elektrisches Signal und die physikalische Größe durch die gesamte elektrische Ladung repräsentiert wird, die durch Integration der Amplitude des elektrischen Signals innerhalb der Zeitspanne vom Beginn der Analyt- Bioelement- Wechselwirkung bis zu dem Zeitpunkt, zu dem die Änderung gegen null tendiert oder die Größe zu einem im Wesentlichen konstanten Wert tendiert, wobei die gesamte elektrische Ladung proportional zu der Konzentration des Analyten ist.

4. Verfahren nach Anspruch 1 oder 2, wobei die physikalische Größe durch die Leitfähigkeit einer Lösung repräsentiert wird, die das System Analyt- Bioelement enthält, und die elektrische Leitfähigkeit gemessen wird, nachdem die gesamte Analytmenge mit dem Bioelement in Wechselwirkung getreten ist, wenn die Leitfähigkeit dazu tendiert, einen angenähert konstanten Wert anzunehmen, wobei der gemessene Wert der Leitfähigkeit proportional ist zu der Konzentration des Analyten.

5. Einrichtung zur Bestimmung der Konzentration eines Analyten unter Verwendung eines Bioelements nach dem Verfahren eines der Ansprüche 1 bis 4, umfassend eine Kammer zur Aufnahme eines Systems Analyt- Bioelement und mit der Kammer verbundene Übertragermittel zur Detektion der Änderung der chemischen, physikalischen und/oder chemo-physikalischen Eigenschaften des Systems Analyt-Bioelement und Umsetzung der Änderungen in eine physikalische Größe, die mit der Konzentration des Analyten in Korrelation gebracht werden kann, wobei die Kammer eine erste Oberfläche umfasst, auf der wenigstens ein Teil der Übertragermittel angeordnet ist, und eine gegenüberliegende zweite Oberfläche, wobei die Tiefe der Kammer, die gleich dem Abstand zwischen den Oberflächen ist, gleich oder kleiner 500 µm ist, ein Bioelement auf wenigstens einer der Oberflächen bereitgestellt wird, **dadurch gekennzeichnet, dass** sie ein plattenähnliches Element umfasst, das auswechselbar mit der Einrichtung verbunden isc und die Oberfläche definiert, auf der das Bioelement angeordnet wird.

6. Einrichtung nach Anspruch 5, wobei die Tiefe zwischen 2 und 200 µm und vorzugsweise zwischen 20 und 100 µm gewählt wird.

7. Einrichtung nach Anspruch 5 oder 6, wobei das Verhältnis zwischen Länge der Kammer und Tiefe der Kammer, die gleich dem Abstand zwischen den Oberflächen ist, zwischen 50 und 500 liegt.

8. Einrichtung nach einem der Ansprüche 5 bis 7, wobei wenigstens eine der Oberflächen von dem Übertrager definiert wird.

9. Einrichtung nach einem der Ansprüche 5 bis 8, wobei das Bioelement auf der dem Übertrager gegenüberliegenden Oberfläche angeordnet ist.

10. Einrichtung nach einem der Ansprüche 5 bis 8, wobei das Bioelement auf der Oberfläche angeordnet ist, die von dem Übertrager definiert wird.

11. Einrichtung nach Anspruch 9, wobei das Bioelement auf der dem Übertrager gegenüberliegenden Oberfläche chemisch und/oder physikalisch immobilisiert ist.

12. Einrichtung nach einem der Ansprüche 5 bis 11, wobei die Oberflächen durch eine Versiegelung getrennt sind, die eine seitliche Oberfläche der Kammer bildet, und die Kammer wenigstens einen Schlauchanschluss umfasst für das Einleiten der Lösung, die den Analyten enthält, in die Kammer bzw. für deren Ausleitung daraus.

13. Einrichtung nach Anspruch 12, wobei die Schlauchanschlüsse Kapillarröhren sind.

14. Einrichtung nach Anspruch 7, wobei die von dem Übertrager definierte Oberfläche der Kammer im Wesentlichen gleich der gegenüberliegenden Oberfläche ist, auf der das Bioelement angeordnet ist.

15. Einrichtung nach einem oder mehreren der Ansprüche 5 bis 14, wobei die Übertragermittel Potentiometerwirkung haben.

16. Einrichtung nach einem oder mehreren der Ansprüche 5 bis 14, wobei die Übertragermittel piezo- elektrisch sind.

17. Einrichtung nach einem oder mehreren der Ansprüche 5 bis 14, wobei die Übertragermittel optisch sind.

18. Einrichtung nach einem oder mehreren der Ansprüche 5 bis 14, wobei die Übertragermittel den Strom oder die Leitfähigkeit messen.

## Revendications

1. Procédé pour déterminer la concentration d'un analyte à l'aide d'un bioélément, comprenant les étapes consistant à :
- introduire dans une chambre (2) comprenant une première surface et une seconde surface (3, 4) disposées l'une en face de l'autre, un échantillon contenant l'analyte, et une quantité d'un bioélément capable d'interagir spécifiquement avec l'analyte, en provoquant un changement dans les caractéristiques chimiques, physiques et/ou chimico-physiques du système analyte-bioélément,
- fournir des moyens formant transducteur (9) sur au moins l'une des surfaces (3, 4) qui convertissent l'interaction analyte-bioélément en une grandeur physique corrélée à la concentration de l'analyte,
**caractérisé en ce que**
- l'interaction analyte-bioélément a lieu à l'intérieur de la chambre (2) ayant une profondeur (H), définie comme étant la distance entre les surfaces (3, 4) qui est choisie de manière à être inférieure ou égale à 500 micromètres,
- **en ce que** la quantité du bioélément choisi est de nature à garantir que tout l'analyte interagit avec le bioélément et
- **en ce que** la concentration de l'analyte est déterminée après que tout l'analyte a interagi avec le bioélément, quand la grandeur a tendance à adopter une valeur approximativement constante qui est proportionnelle à la concentration de l'analyte et qui est aussi indépendante de l'activité du bioélément.

2. Procédé pour déterminer la concentration d'un analyte à l'aide d'un bioélément, comprenant les étapes consistant à :
- introduire dans une chambre (2) comprenant une première surface et une seconde surface (3, 4) disposées l'une en face de l'autre, un échantillon contenant l'analyte,
- fournir sur au moins l'une des surfaces (3, 4) une quantité d'un bioélément capable d'interagir spécifiquement avec l'analyte, en provoquant un changement dans les caractéristiques chimiques, physiques et/ou chimico-physiques du système analyte-bioélément,
- fournir des moyens formant transducteur (9) sur au moins l'une des surfaces (3, 4) qui convertissent l'interaction analyte-bioélément en une grandeur physique corrélée à la concentration de l'analyte,
**caractérisé en ce que**
- l'interaction analyte-bioélément a lieu à l'intérieur de la chambre (2) ayant une profondeur (H), définie comme étant la distance entre les surfaces (3, 4) qui est choisie de manière à être inférieure ou égale à 500 micromètres,
- **en ce que** la quantité du bioélément choisi est de nature à garantir que tout l'analyte interagit avec le bioélément et
- **en ce que** la concentration de l'analyte est déterminée après que tout l'analyte a interagi avec le bioélément, quand la grandeur a tendance à adopter une valeur approximativement constante qui est proportionnelle à la concentration de l'analyte et qui est aussi indépendante de l'activité du bioélément.

3. Procédé selon la revendication 1 ou la revendication 2, dans lequel le changement dans les caractéristiques chimiques, physiques et/ou chimico-physiques du système analyte-bioélément est converti en un signal de courant électrique et la grandeur physique représente la charge électrique totale obtenue par intégration de l'amplitude du signal de courant électrique pendant le laps de temps entre le moment où débute l'interaction analyte-bioélément et le moment où le changement tend vers zéro ou la grandeur tend vers une valeur sensiblement constante, la quantité totale de charge électrique étant proportionnelle à la concentration de l'analyte.

4. Procédé selon la revendication 1 ou la revendication 2, dans lequel la grandeur est représentée par la conductivité électrique d'une solution contenant le système analyte-bioélément, la conductivité électrique étant mesurée après que tout l'analyte a interagi avec le bioélément quand la conductivité tends vers une valeur sensiblement constante, la valeur de conductivité mesurée étant proportionnelle à la concentration de l'analyte.

5. Dispositif pour déterminer la concentration d'un analyte à l'aide d'un bioélément selon le procédé de l'une quelconque des revendications 1 à 4, comprenant une chambre destinée à contenir un système analyte-bioélément et des moyens formant transducteur associés à la chambre pour détecter le changement dans les caractéristiques chimiques, physiques et/ou chimico-physiques du système anatyte-bioélément et convertir les changements en une grandeur physique qui peut être corrélée à la concentration de l'analyte, la chambre comprenant une première surface sur laquelle est disposée au moins une partie des moyens formant transducteur, et une seconde surface opposée, la profondeur de la chambre, qui est égale à la distance entre les surfaces, étant inférieure ou égale à 500 micromètres, un bioélément étant disposé sur au moins l'une des surfaces,
**caractérisé en ce qu'**il comprend un élément en forme de plaque associé au dispositif de façon interchangeable et définissant la surface sur laquelle est disposé le bioélément.

6. Dispositif selon la revendication 5, dans lequel la profondeur est comprise entre 2 et 200 micromètres et est de préférence choisie entre 20 et 100 micromètres.

7. Dispositif selon la revendication 5 ou la revendication 6, dans lequel le rapport entre la longueur et la profondeur de la chambre, qui est égale à la distance entre les surfaces, est compris entre 50 et 500.

8. Dispositif selon l'une quelconque des revendications 5 à 7, dans lequel au moins l'une des surfaces est définie par le transducteur.

9. Dispositif selon l'une quelconque des revendications 5 à 8, dans lequel le bioélément est disposé sur la surface opposée au transducteur.

10. Dispositif selon l'une quelconque des revendications 5 à 8, dans lequel le bioélément est disposé sur la surface définie par le transducteur.

11. Dispositif selon la revendication 9, dans lequel le bioélément est immobilisé chimiquement et/ou physiquement sur la surface opposée au transducteur.

12. Dispositif selon l'une quelconque des revendications 5 à 11, dans lequel les surfaces sont séparées par un joint d'étanchéité définissant une surface latérale de la chambre, le dispositif comprenant au moins un conduit pour l'entrée de la solution contenant l'analyte dans la chambre et pour sa sortie de celle-ci, respectivement.

13. Dispositif selon la revendication 12, dans lequel les conduits sont des conduits capillaires.

14. Dispositif selon la revendication 7, dans lequel l'aire de la surface de la chambre définie par le transducteur est sensiblement égale à l'aire de la surface opposée sur laquelle est disposé le bioélément.

15. Dispositif selon une ou plusieurs des revendications 5 à 14, dans lequel les moyens formant transducteur sont potentiométriques.

16. Dispositif selon une ou plusieurs des revendications 5 à 14, dans lequel les moyens formant transducteur sont piezoélectriques.

17. Dispositif selon une ou plusieurs des revendications 5 à 14, dans lequel les moyens formant transducteur sont optiques.

18. Dispositif selon une ou plusieurs des revendications 5 à 14, dans lequel les moyens formant transducteur sont ampérométriques ou conductimétriques.
